Europäisches Patentamt

European Patent Office

Office européen des brevets

(11) Publication number: **0 478 017 A1**

## EUROPEAN PATENT APPLICATION

(21) Application number: **91121423.7**

(22) Date of filing: **12.06.86**

(51) Int. Cl.⁵: **C07K 15/28**, //C12P21/08, G01N33/68,G01N33/543

This application was filed on 13 - 12 - 1991 as a divisional application to the application mentioned under INID code 60.

(30) Priority: **13.06.85 JP 129037/85**

(43) Date of publication of application: **01.04.92 Bulletin 92/14**

(60) Publication number of the earlier application in accordance with Art.76 EPC: **0 205 177**

(84) Designated Contracting States: **BE CH DE FR GB IT LI NL SE**

(71) Applicant: **Yamasa Shoyu Kabushiki Kaisha 10-1, Araoi-Cho 2-Chome Choshi-Shi Chiba-Ken 288(JP)**

(72) Inventor: **Yazaki, Yoshio 12-4, Kohinata 3-chome Bunkyo-ku, Tokyo-to(JP)**
Inventor: **Sugi, Masahito 2-2, Sakae-cho 2-chome Choshi-shi, Chiba-ken(JP)**
Inventor: **Ishige, Masao 5802, Kasagami-cho Choshi-shi, Chiba-ken(JP)**
Inventor: **Kato, Hirohisa 2-2, Sakae-cho 2-chome Choshi-shi, Chiba-ken(JP)**
Inventor: **Chino, Shinichi 2-2, Sakae-cho 2-chome Choshi-shi, Chiba-ken(JP)**
Inventor: **Kuroda, Manami 2-1, Araoi-cho 2-chome Choshi-shi, Chiba-ken(JP)**

(74) Representative: **Dr. Elisabeth Jung Dr. Jürgen Schirdewahn Dipl.-Ing. Claus Gernhardt P.O. Box 40 14 68 Clemensstrasse 30 W-8000 München 40(DE)**

(54) Monoclonal antibody with specificity for human myosin light chains.

㉗ The present invention provides a monoclonal antibody having specificity for the human myosin light chain from muscle and which also recognizes the common antigenic determinant of both myosin light chains from xenogeneic animal muscle and myosin light chains existing in the human serum of a patient with a muscle disease.

Such an antibody, for the first time, has made possible the establishment of a practical method of diagnosing patients with muscle diseases such as myocardial infarction by immunological assay of myosin light chains.

EP 0 478 017 A1

Field of the Art

The present invention relates to a monoclonal antibody which has specificity for human myosin light chains I or II existing in the human serum of a patient with a muscle disease. Such an antibody is very useful for carrying out a method of immunologically assaying free myosin light chains in the blood.

Prior Art

La Due et al. have reported that glutamic -oxaloacetic transaminase (GOT) activity is raised in the serum of an acute myocardial infarction patient (J. S. La Due et al., Science, 120, 497 (1954)), and thereafter the methods for biochemical diagnosis of myocardial infarction have been successively developed by assaying the activities of the enzymes flowing out into the blood from cardiac cells by ischemic cardiac disorder. Among them, activities of GOT, lactate dehydrogenase(LDH) and creatine phosphokinase (CPK), can be assayed with relative ease, and it has been shown that the enzyme activity in the sera is raised in 90% or more of acute myocardial infarction patients (N. S. Sarensen, Acta. Med. Scand., 174, 725 (1963)). Also, the biochemical diagnostic methods are attracting attention not only for the diagnosis of myocardial infarction, but also as a method for measuring the amount of infarcted cardiac muscle from the standpoint of evaluation of therapy and judgement of prognosis.

However, these biochemical diagnostic methods involve problems such as (1) a problem with respect to specificity of the target enzyme due to the presence of isozymes which have flowed out from other organs, (2) uncertainty of the correlation between the amount of the enzyme flowing out into the blood from the cardiac muscle at the infarcted portion and the extent of myocardial infarction, and (3) loss of the correlation thereof with the amount of infarcted cardiac muscle during the use of a thrombolytic agent such as urokinase or tissue plasminogen activator which has become recently available for therapy of myocardial infarction since these medicines cause the above-mentioned cytoplasmic enzymes to flow out at once into the blood (wash-out effect).

As one of the markers which can be new indexes in an alternative diagnostic method of myocardial infarction for such biochemical diagnostic methods of the prior art, cardiac myosin has attracted considerable attention. A cardiac myosin molecule exists most abundantly (60%) among the constitutive proteins of cardiac cells, is a structural protein with a molecular weight of about 500,000 having the central role of the contraction mechanism of muscles, and has a subunit structure comprising two heavy chains with a molecular weight of 200,000, two light chains I (LCI) with a molecular weight of 27,000 and two light chains II (LCII) with a molecular weight of 20,000. Cardiac myosin light chain or its fragment is considered to reflect directly the degradation process of cardiac cells by ischemia since it is released into the blood with the accompaniment of destruction of cell membranes due to ischemic cardiac disorder. This is evidenced by radioimmunoassay using the rabbit antiserum specific to cardiac myosin light chain (R. Nagai et al., Biochem. Biophys. Res. Commun., 86, 683 (1979)), and it is also reported that cardiac myosin is specifically applicable in a clinical diagnostic method for myocardial infarction (Ryozo Nagai et al., Journal of Internal Medicine of Japan, 70, 16 (1981); Katus et al., Am. J. Cardiol., 54, 964-970 (1984)).

Cardiac myosin light chain has the following specific features as an index substance flowing out into the blood due to ischemic cardiac disorder.

(1) Cardiac myosin exists most abundantly as a structural protein in cardiac cells.

(2) Cardiac myosin has a subunit structure comprising heavy chains and light chains, can readily be dissociated from the myosin molecule by change in pH, etc., the light chain with lower molecular weight being readily released out of the cells.

(3) Cardiac myosin light chain contains only a small amount of thiol groups, is a biochemically stable protein, and also is not susceptible to action by proteolytic enzymes existing in the tissue or blood.

(4) Even in a patient administered with a thrombolytic agent such as urokinase, cardiac myosin light chain is free from the influence of the wash-out effect thereby, and the change in cardiac myosin light chain level in the blood with elapse of time coincides with the pattern of the degradation process of cardiac cells by ischemia.

Regarding methods for carrying out immunological assay of cardiac myosin light chain by the use of antiserum specific to human cardiac myosin light chain, other than those mentioned above, there are reports as enumerated below.

Trahern et al., Am. J. Cardiol., 41, 4, 641-645 (1978).

Khaw et al., Circulation, 58, 1130-1136 (1979).

Katus et al., Circulation, 60 (Suppl. II) 139 (1979).

Further, reports have also been made on methods utilizing monoclonal antibody specific to cardiac myosin as an antibody in immunological assay, as follows.

Haber et al., J. Mol. Cell. Cardiol., 14, Suppl. 3, 139-146 (1982).

Katus et al., Molecular Immunology, 19, 3, 451-455 (1982).

The use of a monoclonal antibody having specificity for human cardiac myosin light chain as an antibody reagent in an immunological assay would have advantages, as compared to the use of an antiserum: (1) if the antibody has high specificity to human cardiac myosin light chain and little cross-reaction with skeletal myosin light chain and if (2) such an antibody with high specificity can be supplied in a large amount and continuously, then it would be more suitable for practical application of the diagnosis of myocardial infarction by assay of cardiac myosin light chain.

On the other hand, also in skeletal muscle disease such as multiple myositis or Duchenne type myodystrophy, skeletal myosin light chain flows out into the blood of a patient similarly as in myocardial disease to become an index of muscle tissue degradation, as clarified by the radioimmunoassay by the use of anti-cardiac myosin light chain antibody with great cross-reactivity with skeletal myosin light chain (Nippon Rinsho, 40, Suppl. in Autumn, 107-111 (1982)). However, its immunological assay has not yet been established.

In the immunological assay of cardiac myosin light chain utilizing a monoclonal antibody of the prior art, there have been involved problems such as that (1) most of the monoclonal antibodies obtained by the use of the isolated and purified cardiac myosin light chain, while exhibiting specificity to the isolated and purified cardiac myosin light chain, will not bind to the cardiac myosin light chains existing in the blood of a human myocardial infarction patient, as confirmed by our experiments, and no such possibility of assay in myosin light chain in human serum has been examined and that (2) since the cardiac myosin light chain used for the standard substance and/or the labeled cardiac myosin light chain is derived from human, it is difficult to prepare such cardiac myosin light chain in order to apply it practically in these immunoassay systems as a diagnostic reagent.

The present invention is intended to solve these problems in providing a monoclonal antibody which does not have the above discussed disadvantages of the prior art and is therefore suitable for carrying out a diagnostic method of myocardial infarction or skeletal muscle disease.

## SUMMARY OF THE INVENTION

We have conducted various studies and as a result successfully obtained monoclonal antibodies which can recognize not only the antigenic determinant of the purified human myosin light chain but also that of myosin light chain existing in the serum of a patient having a muscle disease such as myocardial infarction. Further, by selecting from among these monoclonal antibodies such a monoclonal antibody which can also recognize the antigenic determinant of the myosin light chain of a xenogeneic animal such as hog, dog or cattle, the present invention has been achieved.

More specifically, the monoclonal antibody according to the present invention has specificity for human myosin light chain I prepared (isolated or extracted) from muscle and which also recognizes the common antigenic determinant of both myosin light chains I and/or II prepared (isolated or extracted) from xenogeneic animal muscle and myosin light chains existing in the human serum of a patient with a muscle disease.

According to another embodiment the monoclonal antibody has specificity for human myosin light chain II prepared (isolated or extracted) from muscle and which also recognizes the common antigenic determinant of both myosin light chains II and/or I prepared (isolated or extracted) from xenogeneic animal muscle and myosin light chains existing in the human serum of a patient with a muscle disease.

## BRIEF DESCRIPTION OF THE DRAWINGS

In the drawings:

Fig.1 is a graph showing the standard curves obtained by using the monoclonal antibody of Example 2 in an immuno assay;

Fig.2 is that obtained in Example 4;

Fig.3 is that obtained in Example 5; and

Fig.4 is a graph showing the change with elapse of time in the cardiac myosin light chain content in the serum of the myocardial infarction patient in Example 6.

## DETAILED DESCRIPTION OF THE INVENTION

In the present invention, the term "myosin light chain" refers to light chain I and/or light chain II of cardiac (ventricular or atrial) myosin or fragments thereof, or light chain I, light chain II and/or light chain III of skeletal myosin or fragments thereof.

The expression "monoclonal antibody having specificity for human myosin light chain" refers to a monoclonal antibody having binding ability to light chain I and/or light chain II of cardiac myosin or fragments thereof, or monoclonal antibody having binding ability to light chain I, light chain II and/or light chain III of skeletal myosin or fragments thereof.

The term "xenogeneic animal" refers primarily to mammals other than human being such as monkey, dog, cat, hog, cattle, horse, goat, sheep, rabbit, mouse, rat, guinea pig, hamster and squirrel.

Further, the term "immunological assay" in the description refers comprehensively to the methods based on the principle that the assaying system basically detects the difference in formation of antigen-antibody complex depending on the existing amount of an antigen in a sample by chemical or physical means by utilizing the antigen and the binding ability of an antibody specific thereto and determines the amount of the antigen in the sample with reference to that in a standard sample containing a known amount of the antigen. A large number of such immunological assays have heretofore been developed. For example, assaying systems may be classified broadly into the agglutination reaction method (immunonephelometry), the agglutination inhibition method, the immunodiffusion method, the laser nephelometry, the competitive reaction method (liquid phase or solid phase method), the sandwich assay and the immunometric assay. As assaying systems using labeling substances, depending on the species of the labeling substance, radioimmunoassay (RIA) using radioisotopes such as $^{125}$I, $^{131}$I, $^{3}$H and $^{14}$C; enzyme immunoassay (EIA) using enzymes such as $\beta$-galactosidase, peroxidase, alkaline phosphatase and acetylcholin esterase;

fluorescent immunoassay (FIA) using fluorescent dyes such as umbelliferone, fluorescein, rhodamine, fluorescein isothiocyanate and phycoerythrin; chemiluminescent immunoassay using a chemical luminescent substance such as luminol; metal immunoassay and spin immunoassay have been known. These assaying methods can be applied within the context of the present invention as far as they are based on the above assaying principle and applicable for the assaying system of myosin light chain.

In applying these individual immunological assays, no particular conditions or operations are required. By applying conventional technical considerations of those skilled in the art to the conventional procedure, means and conditions in each method, an assaying system may be constituted by selecting a monoclonal antibody having the above stated properties as an antibody reagent and a myosin light chain from a xenogeneic animal recognized by the above monoclonal antibody as an antigen reagent so as to be adaptable to the cardiac myosin light chain assaying system or the skeletal myosin light chain assaying system. As to details relating to the general techniques of these methods, reference may be made to general reviews and textbooks (for example, Minoru Irie, ed. "Radioimmunoassay" (Kabushiki Kaisha Kodansha, published on April 10, 1974); Minoru Irie, ed. "Radioimmunoassay, second series" (Kabushiki Kaisha Kodansha, published on May 1, 1979); Eiji Ishikawa et al., ed. "Enzyme Immunoassay" 2nd Edition (Kabushiki Kaisha Igaku Shoin, published on December 15, 1982); Nippon Rinsho, 42, Supplemental Vol. in Spring (1984) "Handbook of Clinical Immunology" (Kabushiki Kaisha Nippon Rinshosha, published on March 20, 1984), 1198-1227; H. V. Bunachis et al., "Methods in Enzymology" 70, "Immunochemical Techniques Part A" (Academic Press, 1980); J. J. Langon et al., "Methods in Enzymology" 73, "Immunochemical Techniques Part B", ibid. 74 "Immunochemical Techniques Part C" (Academic Press, 1981).

Assaying systems of the methods typical of these immunological assays are as described below.

① Agglutination reaction method (immunonephelometry):

To a sample, an antibody specific to the antigen to be measured or an antibody insolubilized on a carrier such as erythrocyte or polymer latex particles is added, and the amount of the antigen-antibody complex formed by the antigen-antibody reaction is measured in terms of the increase in turbidity or the degree of light scattering to determine the antigen amount.

② Competitive reaction method:

The antigen in a sample and a predetermined amount of a labeled antigen are subjected to a competitive reaction with an antibody; the unreacted labeled antigen (F) is separated from the labeled antigen (B) bound to the antibody (BF separation); and the label quantity of either one of B and F is measured to determine quantitatively the antigen amount in the sample.

There are the liquid phase method, in which a soluble antibody is used as an antibody and ammonium sulfate, polyethylene glycol and/or a second antibody specific to the above antibody is used for BF separation, and the solid phase method, in which a solid phase antibody is used as an antibody, or a soluble antibody is used as a first antibody and a solid phase antibody is used as a second antibody.

③ Sandwich assay:

The antigen in a sample is allowed to react with a first antibody immobilized on a solid carrier. Further the antigen is allowed to react with a labeled second antibody recognizing the site other than the binding site with the first antibody. The liquid phase is separated from the solid phase, and the label amount in the liquid phase or the solid phase is measured to determine quantitatively the antigen amount in the sample.

As a modification, there is also an indirect labeling method in which, without labeling directly the second antibody, by the use of a second antibody having an antigen different from the antigen to be assayed or a binding member (a compound such as biotin) bound thereto, after completion of the reaction between the second antibody with the antigen to be measured and the first antibody, a labeled material of a third antibody specific to the antigen bound to the second antibody or of a bindable substance (avidin), specific to the binding member is allowed to react with the reaction mixture, and the liquid phase is separated from the solid phase, the label amount in either one of them being measured. As a combination of the binding member and the bindable substance, a biotin-avidin system has been widely employed.

④ Immunometric assay:

The antigen in a sample and a solid phase antigen are subjected to a competitive reaction with a predetermined amount of a labeled antibody; the liquid phase is separated from the solid phase; and the label amount in either one of the phases is measured to quantitate the antigen in the sample.

In the given context, the term "antibody reagent" refers to an antibody specific to the antigen to be assayed used in the immunological assays as described above, such as a soluble antibody, solid phase antibody or labeled antibody, or an antibody having a binding member specific to a labeled material used in the indirect labeling method.

In the given context. the term "antigen reagent" refers to an antigen such as an antigen for a standard solution, solid phase antigen or labeled antigen used in the immunological assays as described above.

Preparations of these reagents are described below.

(1) Preparation of antigen (myosin light chain)

Myosin can be prepared in a conventional manner by extraction from cardiac (ventricular or atrial) muscle or skeletal muscle of an animal bearing myosin light chain having an antigenic determinant common (i.e., immunologically equal) to the human myosin light chain, followed by purification. Extraction of myosin is carried out generally with the use of a salt solution such as 0.2 - 0.6 M potassium chloride under the condition of pH 6.5 - 7.0. The preparation of myosin light chain can be practiced by applying suitably a known method such as ion-exchange chromatography using ion exchanger such as DEAE-Sephadex® A-25, DEAE-Sephadex® A-50; gel filtration chromatography using gel filler such as Sephadex® G-200; affinity chromatography; precipitation method with a chelating agent; urea or guanidine hydrochloride treatment; preparative electrophoresis; dialysis; and high concentration salt solution treatment, (see "Course of Biochemical Experiments 15, Muscle" (Kabushiki Kaisha Tokyo Kagaku Dojin, published on November 25, 1975), 3-12; R. Nagai et al., Biochem. Biophys. Res. Commun., 86, 683 (1979); A. M. Katz et al., Cir. Res., 19, 611-621 (1965); W. T. Perrie et al., Biochem. J., 119, 31-38 (1970)).

In the cardiac myosin light chain assaying system in which a monoclonal antibody according to the present invention is used, any of the cardiac myosin light chain I, the cardiac myosin light chain II derived from a xenogeneic animal and a mixture thereof can be utilized. This is true in the case of the skeletal myosin light chain assaying system.

(2) Preparation of antibody

The preparation of a monoclonal antibody of the invention having the above stated properties can be practiced by applying the known cell fusion method, transformation method with EB virus, etc. The cell fusion method is more suitable for large-scale production of an antibody, and the preparation steps of the antibody of the present invention by way of the cell fusion method are described below. As to details relating to the general techniques of the cell fusion method, reference can be made to the descriptions in textbooks or reports (e.g., see Tatsuo Iwasaki et al., "Monoclonal Antibody-Hybridoma and ELISA" (Kabushiki Kaisha Kodansha, published on February 20, 1983); G. Köhler et al., Eur. J. Immunol., 6, 511-519 (1976); M. Shulman et al., Nature, 276, 269-270 (1978)).

(a) Preparation of antibody-producing cells

The myosin light chain prepared from cardiac or skeletal muscle of animals including human being in the same manner as described above is immunized to a xenogeneic animal, and antibody-producing cells such as spleen cells, lymph node cells or peripheral blood lymphocytes are obtained in a conventional manner from the animal which has acquired immunity.

(b) Preparation of myeloma cells

As myeloma cells, cell lines originated from various animals such as mice, rats, rabbits and humans and easily available to those skilled in the art can be used. The cell line to be used should preferably be drug resistant, not viable in a selective medium but viable after fusion with antibody-producing cells. The cell line most commonly used is an 8-azaguanine resistant cell line, which is defective in hypoxanthine phosphoribosyl transferase and cannot be grown in hypoxanthine-aminopterine-thymidine (HAT) medium. The cell line is also preferably of the so-called non-secretor type which does not secrete immunoglobulin.

Typical examples of myeloma cell lines are $P_3$/x63-Ag 8 (Nature, 256, 495-497 (1975)), $P_3$/x63-Ag 8 $U_1$-($P_3U_1$) (ATCC CRL-1597) (Current Topics in Microbiology and Immunology, 81, 1-7 (1978)), $P_3$/x63-Ag 8•6•5•3 (x63•6•5•3)(ATCC CRL-1580) (J. Immunology, 123, 1548-1550 (1979)), $P_3$/NSI-1-Ag 4-1 (NS-1) (European J. Immunology, 6, 292-295 (1976)), Sp2/0-Ag 14 (SP2) (ATCC CRL-1581) (Nature, 276, 269-270 (1978)) derived from mouse myeloma (MOPC-21) cell line. Rat myeloma cell line such as 210 RCY. Ag 1•2•3 (Y3 Ag 1•2•3) (Nature, 277, 131-133 (1979)), and human myeloma cell line such as U-266-AR$_1$ (Proc. Natl. Acad. Sci. U.S.A., 77, 1158 (1980)), GM1500 (Nature, 288, 448 (1980), and KR-4 (Proc. Natl. Acad. Sci. U.S.A., 79, 6651 (1982)) are also available.

(c) Cell fusion

In cell fusion, myeloma cells suitable for antibody-producing cells are selected. Cell fusion is carried out by mixing $10^7$ to $10^8$ myeloma cells with antibody-producing cells at a mixing ratio of from 1:4 to 1:10 in a medium for culturing animal cells such as Eagle's minimum essential medium (MEM), Dulbecco's modified Eagle's medium (DMEM) and RPMI 1640. For promoting cell fusion, a fusing aid such as a polyethylene glycol (PEG) having an average molecular weight of 1,000 to 6,000, a polyvinyl alcohol or a virus may be used.

(d) Selection of hybridoma in selective medium

Selection of the desired hybridoma from the cells after the cell fusion process is conducted by selective growth in a selective medium. For example, the cells are diluted appropriately with, for example, RPMI 1640 medium containing 15% fetal calf serum (FCS), placed on a microtiter plate to about $10^5$ - $10^6$ cells/well, and a selective medium (e.g., HAT medium) is added to each well, which step is followed by appropriate exchange of the selective medium. For example, when an 8-azaguanine resistant cell line is used as the myeloma cell and a HAT medium as the selective medium, unfused myeloma cells will die on about the 10th day after cultivation, and the antibody-producing cells which are normal cells cannot be grown in vitro for a long term. Accordingly, the cells grown on the 10th to 14th day are all hybridomas.

(e) Screening for anti-myosin light chain antibody-producing hybridomas

A screening for hybridomas producing anti-myosin antibody can be carried out according to, for example, the Enzyme Linked Immunosorbent Assay (ELISA) or the Radioisotope Immunoassay (RIA). For example, a culture supernatant containing hybridomas is added to a 96-well microplate coated with myosin light chain of human or a xenogeneic animal to allow the specific antibody to react therewith, and then the bound specific antibody is allowed to react with an enzyme-labeled anti-immunoglobulin antibody or with a biotinylated anti-immunoglobulin antibody and then with avidin D-enzyme labeled material, which step is followed by color formation with addition of the substrate for the enzyme to each well. Depending on the presence of color formation, the well of the culture supernatant containing the hybridoma producing an antibody specific for the myosin light chain can be judged, whereby screening for hybridoma can be carried out.

By testing the presence of the characteristics of the monoclonal antibody as stated above, the hybridoma producing such an antibody can be finally selected.

(f) Cloning

Cloning of hybridomas can be conducted according to, for example, the limiting dilution method, the soft agar method, the fibrin gel method, and the fluorescence-activated cell sorter method.

(g) Production of antibody

As a method for producing an anti-myosin light chain monoclonal antibody from the anti-myosin light chain antibody-producing hybridoma thus obtained, a conventional cell cultivation method or ascites formation method may be employed.

In the cell cultivation method, the hybridoma is cultured in a medium for culturing animal cells such as RPMI 1640 medium containing 10 to 15% FCS or serum-free medium, and the antibody can be obtained from the culture supernatant.

In the method for recovering the antibody from ascites, after a mineral oil such as pristane(2,6,10,14-tetramethylpentadecane) has been administered intraperitoneally into an animal the major histo-compatibility of which coincides with the hybridoma, the hybridoma is injected intraperitoneally in an amount of about $10^7$ cells. Hybridomas will grow as ascitic tumors within 10 to 18 days to produce antibodies in high concentrations in serum and ascitic fluid.

When purification of the antibody is required, purification can be carried out by suitably selecting and combining methods such as the ammonium sulfate salting-out method, ion exchange chromatography utilizing anion exchanger such as DEAE cellulose, affinity chromatography using Sepharose® 4B having myosin light chain bound thereto or Protein A-Sepharose®, and molecular sieve chromatography.

When the myosin light chain is to be measured in the primary diagnostic method of common muscle disease or in the diagnostic method of the disease in which myocardiac disease and skeletal muscle disease do not concurrently occur, it is possible to use an antibody having cross-reactivity with both of the cardiac myosin light chain and the skeletal myosin light chain.

(3) Preparation of labeling agent

For preparation of a labeled myosin light chain or a labeled anti-myosin light chain monoclonal antibody, the established known method for the label or the label system employed can be applied. For example, in the case of radioisotope labeling, the chloramine T method or the lactoperoxidase method can be applied. In the case of enzyme labeling, the acid anhydride method, the carbodiimide method, the glutaraldehyde crosslinking method, the periodic acid crosslinking method, the maleimide crosslinking method, etc., can be applied.

(4) Preparation of solid phase forming reagent

Also for preparation of the myosin light chain or anti-myosin light chain monoclonal antibody, a carrier suited for the measuring system employed may be selected, and solid phase formation carried out according to the known method.

For example, materials of the carrier include natural polymer derivative carriers such as cyanogen halide activated products of polysaccharides (e.g. cellulose, dextran, starch, dextrin, hydroxyethyl cellulose, p-aminophenoxyhydroxypropyl dextran, agarose, Sephadex®) (see Japanese Patent Publication No. 38543/1970), sodium metaperiodate activated products of polysaccharides (see Japanese Patent Laid-Open Publication No. 756/1983), aminoethylated or aminopropylated products of cellulose or its derivatives (see Japanese Patent Laid-Open Publication No. 42452/1984), mercaptized products of cellulose or its derivatives (see Japanese Patent Laid-Open Publication No. 80558/1983), cyanated products of polysaccharides (see Japanese Patent Publication No. 28031/1974), epichlorohydrin-p-aminophenol treated products of polysaccharides (see Japanese Patent Laid-Open Publication No. 158994/1979), diazotized products of polysaccharide derivatives containing aromatic amino groups (treated with dil. hydrochloric acid, sodium nitrite), cellulose carbonate derivatives, cellulose acetate, natural cellulose (cotton, hemp, wool); polymer or copolymer carriers of ethylene, propylene, styrene, vinyl alcohol, acrylamide, acrylonitrile, acrylic acid, methacrylic acid, acrylic acid ester, methacrylic acid ester, vinyl acetate or maleic anhydride;

or these carriers into which reactive functional groups such as amino, hydroxyl, carboxyl, sulfone, thiol, azide, isocyano groups are introduced.

Further, the carrier may be in the shape of, for example, a tube, test plate, beads, disc, sphere, stick, or latex.

As the solid phase formation method, any of the physical adsorption method, the covalent bond method, the crosslinking method and the entrapping method can be applied. As to details concerning these solid phase formation methods, the known methods for the enzyme immobilization are applicable, and reference may be made to textbooks or reviews such as "Immobilized Enzyme" edited by Ichiro Chibata (published by Kabushiki Kaisha Kodansha, March 20, 1975), pp 9 - 75.

In practical use of a monoclonal antibody according to the invention no special pre-treatment is required for the preparation of the serum sample to be assayed and serum can be provided as such for the assay. For the purpose of stabilizing the antigen-antibody reaction and preventing non-specific reaction, for example, EDTA with a final concentration of 5 to 10 mM may be added.

By using a monoclonal antibody of the present invention for carrying out an immunoassay there are afforded various advantageous effects such as the following. (1) Practical diagnosis of muscle diseases such as myocardial infarction is rendered possible by selection of a monoclonal antibody having specificity not only for the purified human myosin light chain but also for the myosin light chain existing in the serum of a patient with muscle disease such as myocardial infarction. (2) By selection of an antibody which recognizes the common antigenic determinant of myosin light chain of a xenogeneic animal and of a human being, it is possible to employ the myosin light chain of the xenogeneic animal as an antigen reagent in the immunological assay to make possible the supply of a large amount of the antigen reagent. These beneficial effects afforded by this invention have, for the first time, made possible the establishment of a practical method of diagnosing patients with muscle diseases such as myocardial infarction by immunological assay of myosin light chains.

Example 1 (Preparation of monoclonal antibody specific to myosin light chain)

Human ventricular myosin light chain (1 mg/ml) prepared according to the known method (Biochem. J., 113, 31-38 (1970)) was dissolved in a physiological salt solution and mixed with complete Freund's adjuvant in a ratio of 1:1 to prepare an emulsion. The emulsion was administered intraperitoneally into a BALB/c mouse (female, 6 weeks old) every two weeks to amount to 50 $\mu$g/head, and finally 10 - 30 $\mu$g were administered intravenously.

Three days after the final immunization, spleen cells were taken out of the mouse and washed with MEM. Mouse myeloma $P_3U_1$ was washed with MEM and mixed with the spleen cells in a ratio of 1:10. After centrifugation, 1 ml of 50% PEG 1,000 MEM solution was gradually added to a pellet thus obtained to carry out cell fusion. Further, the MEM solution was gradually added to obtain a final quantity of 10 ml. Again, centrifugation was conducted, and the pellet was suspended in RPMI 1640 medium containing 10% FCS to 3 x $10^4$ cells/0.1 ml as $P_3U_1$ and plating on a 96-well microplate in 0.1 ml/well.

One day later, aliquots each of 0.1 ml of HAT medium were added, and, thereafter every 3 - 4 days, half of the medium was renewed with fresh HAT medium. Each 50 $\mu$l of the supernatant in the well in which growth of hybridoma was recognized was added to a 96-well microplate previously coated with human ventricular myosin light chain. By using biotinylated anti-mouse IgG immunoglobulin (produced by Vector Co.) as the biotinylated anti-immunoglobulin antibody, avidin D-peroxidase (produced by Vector Co.) as the avidin-enzyme conjugate, hydrogen peroxide as the substrate, and 4-aminoantipyrine plus phenol as the chromogenic agent, according to the ELISA method as described above, the hybridoma producing an antibody which reacts with human ventricular myosin light chain was selected and cloned by limiting dilution.

For the monoclonal antibodies produced by the hybridomas obtained, subclasses of the antibodies were determined, and the tests were conducted for cross-reactivity with myosin light chains prepared from human, dog, hog and cattle, and with the ventricular myosin light chains in the serum of a myocardial infarction patient. The results are shown in Tables 1, 2 and 3.

Determination of subclasses was conducted by the use of a MONOABID EIA KIT (supplied by ZYMED Co.) after adding each monoclonal antibody into a 96-well microplate coated with ventricular myosin light chain and blocking with PBS containing 1% bovine serum albumin (BSA).

The index for specificity is represented in terms of relative binding ratio, where the binding ability of the MLM-527 antibody bound to human ventricular myosin light chain is defined as 100, ( + + + ) indicating the rank of 100 to 80%, ( + + ) 80 to 50%, ( + ) 50 to 10% and (-) 10 to 0%.

Preferable examples of antibodies according to the present invention include MLM-508, MLM-520, MLM-527, MLM-536, MLM-544, MLM-162, MLM-515 and MLM-576.

Table 1  ANTIBODIES REACTIVE WITH HUMAN VENTRICULAR MYOSIN LIGHT CHAIN I

| Mono-clonal antibody | Sub-class | Human myosin light chain | | | | Canine myosin light chain | | | Bovine ventri-cular myosin light chain | Porcine ventri-cular myosin light chain |
|---|---|---|---|---|---|---|---|---|---|---|
| | | Ventri-cular light chain I | Ventri-cular light chain II | Patient serum light chain | Skeletal muscle | Ventri-cular light chain I | Ventri-cular light chain II | Skeletal muscle | | |
| MLM-9 | $IgG_1,\kappa$ | + + | - | - | - | N.D. | + + | - | + | - |
| MLM-11 | $IgG_1,\kappa$ | + + | - | - | - | + | + + | - | N.D. | - |
| MLM-25 | $IgG_1,\kappa$ | + +. | - | - | - | N.D. | + + | - | + | - |
| MLM-102 | $IgG_1,\kappa$ | + + | - | - | + | + + | + | N.D. | + | - |
| MLM-106 | $IgG_1,\kappa$ | + + + | - | N.D. | + + | + + + | + + | + | + + | + + |
| MLM-108 | $IgG_1,\kappa$ | + + | - | N.D. | + | + | + | N.D. | + | - |
| MLM-110 | $IgG_{2a},\kappa$ | + + | - | N.D. | + | + + | + + | N.D. | + | - |
| MLM-111 | $IgG_{2a},\kappa$ | + + | - | - | + + | + + | + + | N.D. | + | + + |
| MLM-117 | $IgG_1,\kappa$ | + + + | - | N.D. | + | + + | + + | N.D. | + | - |
| MLM-123 | $IgG_1,\kappa$ | + + + | + | - | + | + + | + + | + | + + + | + + + |
| MLM-136 | $IgG_{2a},\kappa$ | + + + | - | + | + | + + | + + | - | + + + | + |
| MLM-142 | N.D. | + + | + | N.D. | + | + | + + | - | N.D. | - |
| MLM-146 | $IgG_{2a},\kappa$ | + + + | - | N.D. | + + | + + | + + | N.D. | + | + + |
| MLM-147 | $IgG_1,\kappa$ | + + | - | N.D. | - | + + | + | N.D. | + | + + |
| MLM-202 | $IgG_1,\kappa$ | + + | - | N.D. | + + | + + | + + | - | N.D. | + + + |
| MLM-208 | $IgG_1,\kappa$ | + + | - | - | + | + + | + + | - | N.D. | + + |

EP 0 478 017 A1

Table 1 (continued)

| Mono-clonal antibody | Sub-class | Human myosin light chain | | | | Canine myosin light chain | | | Bovine ventri-cular myosin light chain | Porcine ventri-cular myosin light chain |
|---|---|---|---|---|---|---|---|---|---|---|
| | | Ventri-cular light chain I | Ventri-cular light chain II | Patient serum light chain | Skeletal muscle | Ventri-cular light chain I | Ventri-cular light chain II | Skeletal muscle | | |
| MLM-321 | IgG$_1$,κ | +++ | . | . | + | ++ | ++ | + | N.D. | +++ |
| MLM-322 | IgG$_1$,κ | ++ | + | . | ++ | ++ | ++ | . | N.D. | +++ |
| MLM-404 | IgG$_1$,κ | ++ | . | N.D. | . | N.D. | +++ | N.D. | N.D. | +++ |
| MLM-407 | IgG$_{2a}$,κ | +++ | . | . | ++ | N.D. | +++ | . | N.D. | +++ |
| MLM-507 | IgG$_{2a}$,κ | ++ | . | + | ++ | ++ | ++ | + | N.D. | +++ |
| MLM-508 | IgG$_{2a}$,κ | ++ | + | ++ | + | N.D. | +++ | . | N.D. | +++ |
| MLM-510 | IgG$_{2b}$,κ | + | . | . | + | ++ | ++ | . | N.D. | ++ |
| MLM-516 | IgM | ++ | . | N.D. | + | ++ | + | . | N.D. | ++ |
| MLM-520 | IgG$_1$,κ | ++ | . | ++ | + | N.D. | +++ | + | N.D. | +++ |
| MLM-524 | IgG$_{2a}$,κ | +++ | . | N.D. | ++ | +++ | ++ | ++ | N.D. | +++ |
| MLM-527 | IgG$_{2b}$,κ | +++ | . | +++ | +++ | ++ | +++ | +++ | N.D. | +++ |
| MLM-536 | IgG$_{2a}$,κ | ++ | . | ++ | + | +++ | ++ | +++ | N.D. | +++ |
| MLM-544 | IgG$_{2b}$,κ | ++ | . | +++ | + | N.D. | +++ | . | N.D. | +++ |
| MLM-567 | IgG$_1$,κ | ++ | . | N.D. | ++ | ++ | ++ | + | N.D. | +++ |
| MLM-571 | IgG$_{2a}$ | ++ | . | N.D. | + | N.D. | ++ | N.D. | N.D. | ++ |
| MLM-575 | IgG,κ | ++ | . | . | ++ | N.D. | ++ | . | N.D. | +++ |

EP 0 478 017 A1

Table 2  ANTIBODIES REACTIVE WITH HUMAN CARDIAC MYOSIN LIGHT CHAIN II

| Mono-clonal antibody | Sub-class | Human myosin light chain | | | | Canine myosin light chain | | | Bovine ventri-cular myosin light chain | Porcine ventri-cular myosin light chain |
|---|---|---|---|---|---|---|---|---|---|---|
| | | Ventri-cular light chain I | Ventri-cular light chain II | Patient serum light chain | Skeletal muscle | Ventri-cular light chian I | Ventri-cular light chain II | Skeletal muscle | | |
| MLM-105 | N.D. | - | + + | N.D. | + | - | + | - | N.D. | - |
| MLM-113 | IgM,κ | - | + + + | N.D. | + + + | - | + + + | + + + | + | - |
| MLM-132 | N.D. | - | + + | N.D. | - | - | - | - | N.D. | - |
| MLM-137 | N.D. | - | + + | N.D. | + | - | - | - | N.D. | - |
| MLM-162 | IgG$_{2a}$,κ | + | + + | N.D. | + + | - | + + | N.D. | + | - |
| MLM-189 | IgM | + | + + | N.D. | + | + | + + | + | + | - |
| MLM-515 | IgG$_1$,κ | - | + + | N.D. | + + | - | + + | + + | N.D. | - |
| MLM-576 | IgG$_1$,κ | - | + + + | N.D. | + + + | - | + + + | + + | N.D. | - |
| MLM-582 | IgM | - | + + | N.D. | + + | - | + + | + + | N.D. | - |

EP 0 478 017 A1

Table 3     ANTIBODIES REACTIVE WITH HUMAN CARDIAC MYOSIN LIGHT CHAIN I, LIGHT CHAIN II AND SKELETAL MYOSIN LIGHT CHAIN

| Mono-clonal antibody | Sub-class | Human myosin light chain | | | | Canine myosin light chain | | | Bovine ventri-cular myosin light chain | Porcine ventri-cular myosin light chain I |
|---|---|---|---|---|---|---|---|---|---|---|
| | | Ventri-cular light chain I | Ventri-cular light chain II | Patient serum light chain | Skeletal muscle | Ventri-cular light chain I | Ventri-cular light chain II | Skeletal muscle | | |
| MLM-33 | IgM,κ | + | + | N.D. | + | + + | + + | + + | + | - |
| MLM-41 | IgM,κ | + + + | + + + | N.D. | + + | + + | + + | + | + | - |
| MLM-124 | N.D. | + + | + + + | N.D. | + + | + | + + | + + | + + | - |
| MLM-138 | IgG$_1$,κ | + + | + + | N.D. | + + + | + + | + + | + + | + + | - |
| MLM-162 | IgM,κ | + + | + + + | N.D. | + + | + | + + | + + | + | - |
| MLM-205 | N.D. | + + | + + | N.D. | + | + | + + | + | + + | - |
| MLM-227 | N.D. | + | + | N.D. | + | + | + | - | + | - |
| MLM-238 | N.D. | + | + | N.D. | + | + | + | + | + | - |
| MLM-258 | N.D. | + | + | N.D. | + | + | + | - | N.D. | + + + |

Reference Example 1 (Preparation of porcine ventricular myosin light chain)

The ventricular muscle of a porcine heart was cut into suitable pieces, which were washed with 10-fold weight of a 50 mM phosphate buffer containing 1 mM EDTA (pH 6.85). To the tissue pieces was added a 0.6 M potassium chloride - 0.15 M phosphate buffer (pH 6.85, containing 1 mM EDTA and 3 mM potassium pyrophosphate), and the mixture was stirred for 15 minutes to extract myosin. Then, 13-fold weight of a 1 mM EDTA solution was added and the mixture was subjected to filtration. To the filtrate was added 24-fold weight of a 1 mM EDTA solution, and the mixture was left to stand at 4°C overnight. The supernatant was removed by aspiration, and the precipitate was centrifuged at 8,500 r.p.m. at 4°C for 20 minutes. Thus, the precipitate was recovered and weighed. Two-fold amount of a 20 mM Tris-hydrochloride buffer (pH 7.5) containing 1 M potassium chloride, 2 mM EDTA, 40 mM adenosine triphosphate and 10 mM magnesium chloride and an equal amount of distilled water were added, and the mixture was homogenized twice under ice-cooling. After ultra centrifugation at 100,000 xg for 1 hour, 9-fold amount of a 1 mM EDTA solution was added to the supernatant and, with addition of one drop of mercaptoethanol, the mixture was left to stand at 4°C overnight. The precipitate was separated by centrifugation at 8,500 r.p.m. at 4°C for 20 minutes, and to the precipitate obtained was added guanidine hydrochloride to a final concentration of 5 M, after which mercaptoethanol was further added. The mixture was stirred at room temperature for 3 hours and then an equal amount of chilled water and 4-fold amount of ethanol were added, after which centrifugation was carried out at 10,000 r.p.m. at 4°C for 20 minutes. The supernatant was dialyzed several times against a chilled 1 mM EDTA solution, and the dialyzed inner solution was recovered to obtain 100 mg of porcine ventricular myosin light chain.

Reference-Example 2 (Purification of porcine ventricular myosin light chain I)

After the anti-cardiac myosin light chain II monoclonal antibody MLM 515 was purified by DEAE cellulose column chromatography, 100 mg of the antibody was bound to 5 ml of Affigel® 10 (produced by Bio Rad Co.) (binding percentage: 42%) and the gel, after blocking with 1 M ethanolamine, was placed in a column and washed with a 0.2 M glycine hydrochloride buffer (pH 2.5) and a phosphate buffered physiological saline (PBS). The column was loaded with 11 mg of the porcine ventricular myosin light chain prepared in Reference Example 1, which was then eluted with PBS to give 3.7 mg of a purified sample of porcine ventricular myosin light chain I.

Example 2

(1) Preparation of [$^{125}$I]-labeled monoclonal antibody ([$^{125}$I]-antibody)

The anti-human cardiac myosin light chain I monoclonal antibody MLM 508 was purified from mouse ascites by ammonium sulfate fractionation, DEAE cellulose ion exchange column chromatography and Ultrogel® AcA 44 (LKB Co.) gel filtration column chromatography.

The purified antibody was made into a 1 mg/ml solution, of which 40 $\mu$l was $^{125}$I-labeled with 200 $\mu$Ci of sodium iodide ([$^{125}$I]) according to the chloramine T method (Hunter et al., Nature, 194, 495-496 (1962)). This was separated from free radioactive sodium iodide by Sephadex® G25 column chromatography to provide the [$^{125}$I]-antibody.

(2) Preparation of antibody coated tube

The anti-human cardiac myosin light chain I monoclonal antibody MLM 544 was purified in the same manner as described above. The purified antibody was dissolved in PBS (pH 7.4) to 50 $\mu$g/ml, and the solution was added in aliquots each of 0.5 ml into a polystyrene tube and left to stand at 4°C overnight to cause coating. The solution was removed from the tube, and PBS containing 1% BSA and 0.1% sodium azide was added thereto. The solution was then left to stand at room temperature for 2 hours to cause blocking. The BSA solution was removed, and the tube was washed twice with distilled water, freeze-dried and stored in dry state at 4°C before use.

(3) Assay

The standard solutions of the purified human ventricular myosin light chain I or the purified porcine ventricular myosin light chain I were respectively prepared by stepwise dilution with 1% BSA-containing PBS from 1,000 ng/ml to 1 ng/ml. The antibody coated tube was charged with 200 $\mu$l each of the standard solutions, and 250 $\mu$l each of the [$^{125}$I]-antibody diluted to 50,000 c.p.m./250 $\mu$l was then added.

After incubation at room temperature for 16 hours, the supernatant was removed by an aspirator and, after washing twice with distilled water, the radioactivity dose of each tube was measured by a gammacounter.

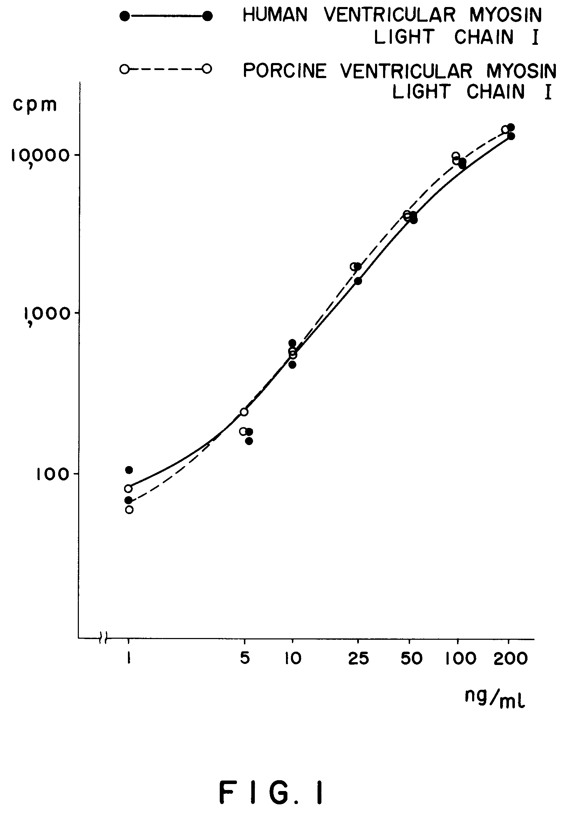

As a result, as shown in FIG. 1, substantially the same standard curve could be prepared for the standard solution of the human ventricular myosin light chain I and the standard solution of the porcine ventricular myosin light chain I from 1 ng/ml to 100 ng/ml.

Thus, it has been clarified that, if multiplied by an appropriate factor, the amount of the human ventricular myosin light chain I can be calculated from the standard curve of the porcine ventricular myosin light chain I.

Example 3

50 $\mu$l each of serum samples collected from 21 healthy individuals and 9 acute myocardial infarction patients was added into an antibody coated tube in the same manner as described above, and 1% BSA-containing PBS was added, which step was followed by addition of 250 $\mu$l of a [$^{125}$I]-antibody solution. After incubation at room temperature for 16 hours, the supernatant was removed by an aspirator, and the tube was washed twice with distilled water and then subjected to measurement of radioactivity by means of an autogammacounter. On the basis of the standard curve prepared with the porcine ventricular myosin light chain I as the standard substance, the myosin light chain in the serum was quantitatively determined.

As a result, the myosin light chain content in the serum sample of a healthy individual was found to be 0.62 ± 0.67 ng/ml. On the other hand, in an acute myocardial infarction patient, it was found to be as high as 11.7 ± 14.3 ng/ml on the day of stroke, reaching a peak on 3 to 6 days after stroke. The value at the peak was 23.3 ± 13.8 ng/ml, and the content was thereafter reduced in many cases until it returned to normal value from one to two weeks later.

Example 4

(1) Preparation of biotinyl antibody

The anti-human cardiac myosin light chain I monoclonal antibody MLM 508 was purified in the same manner as in Example 2 by DEAE cellulose ion exchange chromatography and Ultrogel® AcA 44 gel filtration column chromatography, and this was dialyzed against an aqueous 0.1 M sodium hydrogencarbonate solution, and then the dialyzed inner solution was diluted with the same aqueous solution to an antibody concentration of 1 mg/ml. 0.75 ng of N-hydroxysuccinylbiotin was dissolved in 1 ml of dimethylformamide, and then 0.1 ml of the solution was added into 1 ml of an antibody solution and mixed therewith, and the mixture was left to stand at room temperature for 4 hours. The mixture was dialyzed against PBS containing 0.1% sodium azide to prepare a biotinylated antibody as a PBS solution containing 0.1% BSA and 0.1% sodium azide.

(2) Assay

The antibody coated tube prepared in the same manner as in Example 2 was charged with 100 $\mu$l each of the standard solutions of the human cardiac myosin light chain I or the porcine cardiac myosin light chain I respectively prepared by stepwise dilution with 1% BSA-containing PBS from 1,000 ng/ml to 1 ng/ml and with 150 $\mu$l each of PBS containing 1% BSA and 0.1% sodium azide. After incubation at room temperature for 1.5 hours, the tube was washed twice with PBS. 250 $\mu$l of the diluted biotinylated antibody was added into each tube, which was then left to stand at room temperature for 1.5 hours and washed twice with PBS. 250 $\mu$l of a solution of avidin D-peroxidase (produced by Vector Co.) dissolved in a PBS solution containing 1% BSA and 0.01% sodium ethylmercurithiosalicylate was added into each tube, and the tube was left to stand at room temperature for 15 minutes and washed 3 times with PBS. A chromogenic agent was prepared by dissolving 2 mg of o-phenylenediamine and 50 $\mu$l of a 340 mM hydrogen peroxide solution in 10 ml of a citrate buffer (pH 5.0), and 1 ml of the chromogenic agent was added into each tube. After incubation at room temperature for 20 minutes, the reaction was stopped with addition of 1 ml of 1N sulfuric acid, and absorbance of each tube solution at O.D. 490 nm was measured.

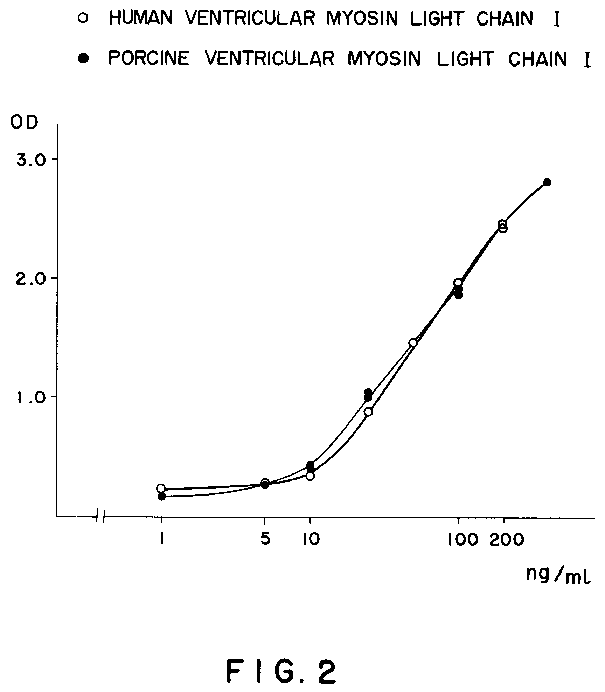

As a result, as shown in FIG. 2, substantially the same standard curve could be prepared for the standard solution of the human ventricular myosin light chain I and the standard solution of the porcine ventricular myosin light chain I from 1 ng/ml to 100 ng/ml.

Example 5

14

Similarly as in Reference Example 1, myosins were extracted from hog, cattle, dog and rat ventricular muscles, and each myosin light chain was isolated by 5 M guanidine hydrochloride treatment. Next, each of these samples was diluted stepwise with PBS containing 1% BSA to prepare standard solutions, and 25 $\mu$l thereof was added into the same antibody coated tube as prepared in Example 2, and then incubated at room temperature overnight with addition of 75 $\mu$l of PBS and 250 $\mu$l of the same [$^{125}$I]-antibody as in Example 2. After removal of the supernatant and washing, the radioactivity dose of each tube was measured by an autogammacounter.

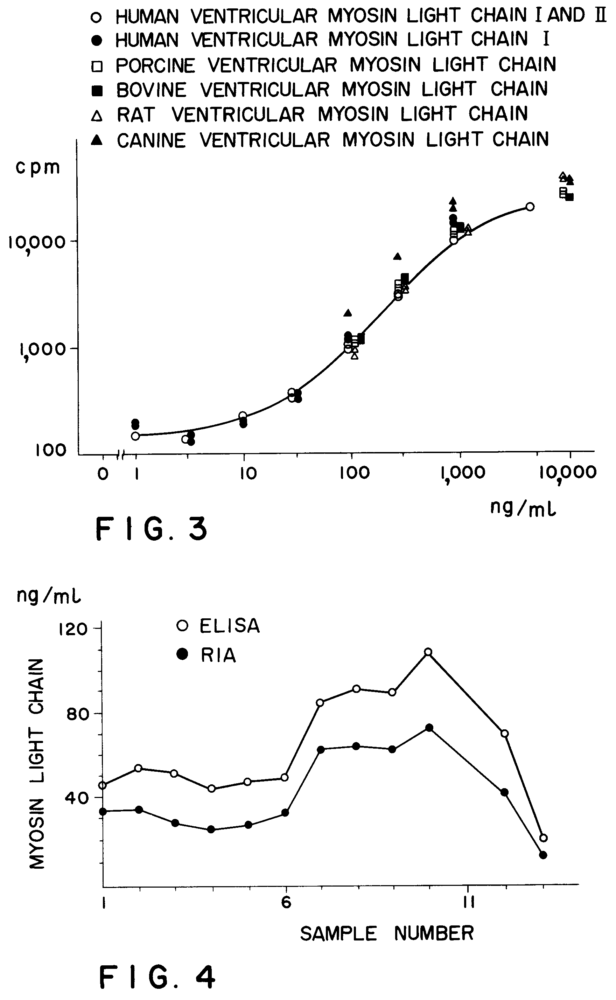

The results are as shown in FIG. 3. It was confirmed that the standard curves obtained from the standard solutions of the respective ventricular myosin light chains of hog, cattle, dog and rat coincide substantially with that of the human ventricular myosin light chain, and therefore the cardiac myosin light chains of these xenogeneic animals could be used as the standard substances in measurement of human cardiac myosin light chain.

Example 6

The serum of an acute myocardial infarction patient was sampled immediately after stroke and thereafter with elapse of time, and assay of cardiac myosin light chain was conducted according to the RIA sandwich method of Example 2 and the ELISA method of Example 4.

The results are as shown in FIG. 4. Although the assayed value obtained by the ELISA method is slightly higher than that obtained by the RIA sandwich method, good correlation was exhibited between both values (correlation coefficient 0.92).

Thus, it has been clarified that both methods are useful for diagnosis of myocardial infarction.

**Claims**

1.  A monoclonal antibody which has specificity for human myosin light chain I prepared (isolated or extracted) from muscle and which also recognizes the common antigenic determinant of both myosin light chains I and/or II prepared (isolated or extracted) from xenogeneic animal muscle and myosin light chains existing in the human serum of a patient with a muscle disease.

2.  A monoclonal antibody which has specificity for human myosin light chain II prepared (isolated or extracted) from muscle and which also recognizes the common antigenic determinant of both myosin light chains II and/or I prepared (isolated or extracted) from xenogeneic animal muscle and myosin light chains existing in the human serum of a patient with a muscle disease.

3.  A monoclonal antibody according to claim 1 or 2 wherein the human myosin light chain is a cardiac myosin light chain, and the xenogeneic animal myosin light chain is also a cardiac myosin light chain.

4.  A monoclonal antibody according to claim 1 or 2, wherein the human myosin light chain is a skeletal myosin light chain, and the xenogeneic animal myosin light chain is also a skeletal myosin light chain.

5.  A monoclonal antibody according to claim 1 or 2, wherein an immunogen for use in the preparation of antibody-producing cells used to produce the antibody is human myosin light chains prepared (isolated or extracted) from human muscle.

6.  A monoclonal antibody according to claim 1 or 2, wherein antibody-producing cells used to produce the antibody are derived from a mouse or a rat.

7.  A monoclonal antibody according to claim 1 or 2, wherein the xenogeneic animal is a canine, bovine, porcine, simian, feline, equine, caprine, ovine, or leporid.

8.  A monoclonal antibody according to claim 7, wherein the xenogeneic animal is a monkey, dog, cat, hog, cattle, horse, goat, sheep, rabbit, mouse, rat, guinea pig, hamster or squirrel.

9.  A monoclonal antibody according to claim 1 or 2, wherein the human myosin light chain is a cardiac myosin light chain, and the xenogeneic animal myosin light chain is a skeletal myosin light chain.

15

FIG. 1

○ HUMAN VENTRICULAR MYOSIN LIGHT CHAIN Ⅰ

● PORCINE VENTRICULAR MYOSIN LIGHT CHAIN Ⅰ

FIG. 2

○ HUMAN VENTRICULAR MYOSIN LIGHT CHAIN I AND II
● HUMAN VENTRICULAR MYOSIN LIGHT CHAIN I
□ PORCINE VENTRICULAR MYOSIN LIGHT CHAIN
■ BOVINE VENTRICULAR MYOSIN LIGHT CHAIN
△ RAT VENTRICULAR MYOSIN LIGHT CHAIN
▲ CANINE VENTRICULAR MYOSIN LIGHT CHAIN

F I G. 3

F I G. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4 ) |
|---|---|---|---|
| A,D | JOURNAL OF MOLECULAR AND CELLULAR CARDIOLOGY vol. 14, no. 3, 1982, pages 139 - 146; E. HABER ET AL.: 'Detection and quantification of myocardial cell death: application of monoclonal antibodies specific for cardiac myosin.' * page 139, line 1 - page 141, column 2, line 8 * | 1-9 | C07K15/28 //C12P21/08 G01N33/68 G01N33/543 |
| A | EP-A-0 131 834 (YAMASA SHOYU KABUSHIKI KAISHA) * the whole document * | 1 | |
| | | | TECHNICAL FIELDS SEARCHED (Int. Cl.4 ) |
| | | | C07K G01N |

The present search report has been drawn up for all claims

| Place of search | Date of completion of the search | Examiner |
|---|---|---|
| THE HAGUE | 29 JANUARY 1992 | HITCHEN C.E. |

EPO FORM 1503 03.82 (P0401)